Europäisches Patentamt

European Patent Office

Office européen des brevets

(11) Publication number: **0 306 614 B1**

# EUROPEAN PATENT SPECIFICATION

(45) Date of publication of patent specification: **23.09.92**   (51) Int. Cl.5: **C07C 273/04**

(21) Application number: **88107081.7**

(22) Date of filing: **03.05.88**

(54) Improvements of processes and plants for the production of urea.

(30) Priority: **15.05.87 CH 1871/87**

(43) Date of publication of application:
**15.03.89 Bulletin 89/11**

(45) Publication of the grant of the patent:
**23.09.92 Bulletin 92/39**

(84) Designated Contracting States:
**AT DE ES FR GB GR IT NL**

(56) References cited:
**DE-A- 1 468 245**
**US-A- 3 824 283**
**US-A- 4 256 662**

(73) Proprietor: **AMMONIA CASALE S.A.**
**Via della Posta 4**
**CH-6900 Lugano(CH)**

(72) Inventor: **Zardi, Umberto**
**Via Lucino 57**
**CH-6932 Breganzona(CH)**
Inventor: **Pagani, Giorgio**
**Salita dei Frati 13**
**CH-6900 Lugano(CH)**

(74) Representative: **Incollingo, Italo**
**AMMONIA CASALE S.A. Via della Posta 4**
**CH-6900 Lugano(CH)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

**Description**

1. Field of the invention

This invention relates to improvements to processes and plants for the production of urea, whereby ammonia and carbon dioxide are converted and the urea solution leaving the reactor is stripped with feed CO2.

2. Description of Prior Art

Processes and plants of this type are widely used and have been remarkably successful, one reason being that besides having a single stripping stage with CO2, they also have:
- a relatively low synthesis pressure (for example, about 140 bar);
- a high degree of depuration of the urea solution directly in the stripper which generally operates at the same synthesis pressure;
- a limited number of downstream stages for finishing the solution (for example stage at 3.5÷4 bar, flash at 1 bar, vacuum and prilling sections).
Even with these undeniable advantages, the systems in question are not without drawbacks, particularly in respect of the consumption of process vapour which is certainly not low (for example 900 kg/t), and which, owing to the increase in the cost of energy due for example to the oil crisis, has become so significant as to constitute a handicap where continuing operation of existing plants is concerned. Numerous (and sometimes brilliant) attempts have been made to improve process efficiency but in almost all cases no concrete results have been achieved to date in the sense that more particularly energy consumption has not been reduced by any appreciable amount.

Moreover, owing to the low NH3/CO2 ratio in the reactor, conversion yields in the outlet reactor are low, thus making further developments more difficult.

The purpose of this invention is to improve the above systems for the production of urea, so as to achieve a drastic reduction in vapour consumption and more particularly to permit a simple and easy modernization of existing plants.

This and other aims are now surprisingly achieved by the addition of at least one stage selected from the group comprising: a mean pressure distillation stage using substantially recovery heat, preferably with double effect, and a pre-distillation stage upstream of the existing stripper.

The characteristics of the various embodiments are described in the claims, while the various aspects and advantages of the invention will be better illustrated by the description of the figures and by the attached tables.

PRIOR ART: STRIPPING PROCESS WITH THE AID OF CO2

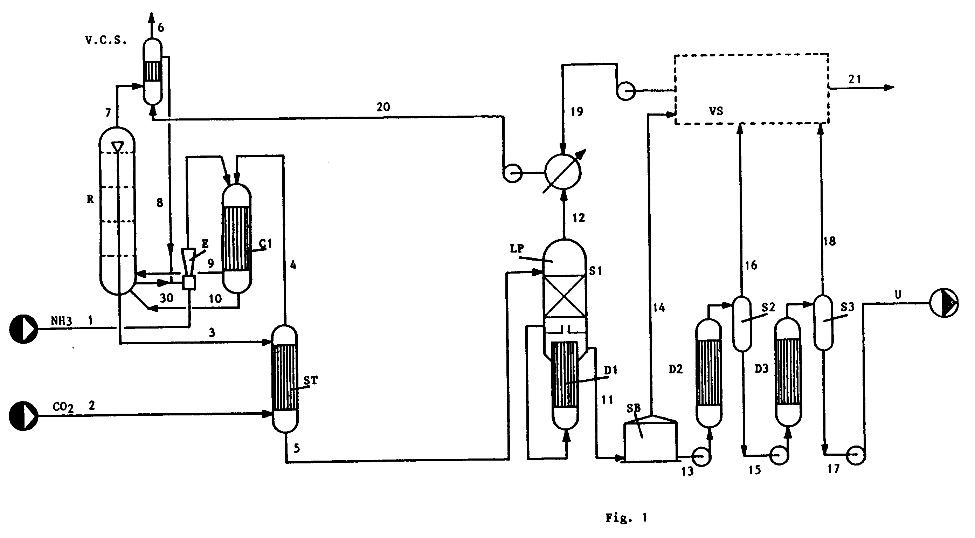

Figure 1 shows the simplified scheme of the conventional process. R indicates the reactor fed with NH3 from line 1, through the ejector, condenser C1 and line 10, and with CO2 from line 2, through stripper ST, condenser C1 and line 9.

The urea solution leaving reactor R flows by gravity (line 3) into stripper ST where the majority of carbamate and of free NH3 are stripped by the feed CO2 (line 2) and recycled to the reactor (line 4), after partial condensation in the film carbamate condenser C1, producing vapour at, for example, 4.5 bar abs.

From condenser C1, the recycle carbamate solution (8) plus a portion (30) of solution taken from the bottom of the reactor itself are sent to reactor R through ejector E.

The driving agent is the feed NH3 (1). The residual vapours leaving from the top 7 of reactor R are sent to the scrubber HP (V.C.S.), where they are scrubbed with the return carbamate solution (20) and subsequently pass into a recovery section known per se and not described.

The solution leaving stripper ST is expanded directly in stage L.P., operating, for example, at about 3.5 bar, where it is distilled in D1 with vapour at, for example, 4.5 bar. Subsequently, it is expanded into the flash tank SB at 1 bar and from here goes into the vacuum distillers D2 and D3. Each distiller D1, D2, D3 is coupled to a separator S1, S2, respectively S3. The urea solution leaving S3 is sent to a crystallization section (known per se), while the vapours are condensed in a vacuum section VS to which also flow the vapours from tank SB.

Conventionally, the formation water is removed from 21 while the solution containing NH3 and CO2 is recycled to stage LF through 19.

For both stripper ST and the second vacuum distiller D3 the vapour used is at 20 bar, whereas recovery

2

vapour at 4.5 bar is used for the other apparatus.

The NH3 recovery section and the vacuum section are of the conventional type and they are not described here.

The typical operating conditions of the process are:
- Mol. ratio NH3/CO2 in the reactor : 2.8
- Mol. ratio H2O/CO2 in the reactor : 0.4
- Reactor yield : 57%
- Temperature at reactor outlet : $183 \div 185\,^\circ$C
- Reactor pressure : 141 bar
- Steam consumption at 20 bar : 1000 kg/t
- Export recovery steam at 4.5 bar : 350 kg/t.

The data relating to the conventional process are shown in Table 1.

SUMMARY OF THE INVENTION

Modernization of the conventional process

It has been found that a first improvement in energy terms in process efficiency can be obtained by adding a mean pressure (= 20 bar) stage in which the distillation heat is provided by the recovery heat/steam, while the vapours obtained are condensed in the first vacuum distiller, thus supplying an important part of the heat necessary for distillation.

Distillation heat can be obtained either by directly condensing the carbamate or with steam at 6 bar at least.

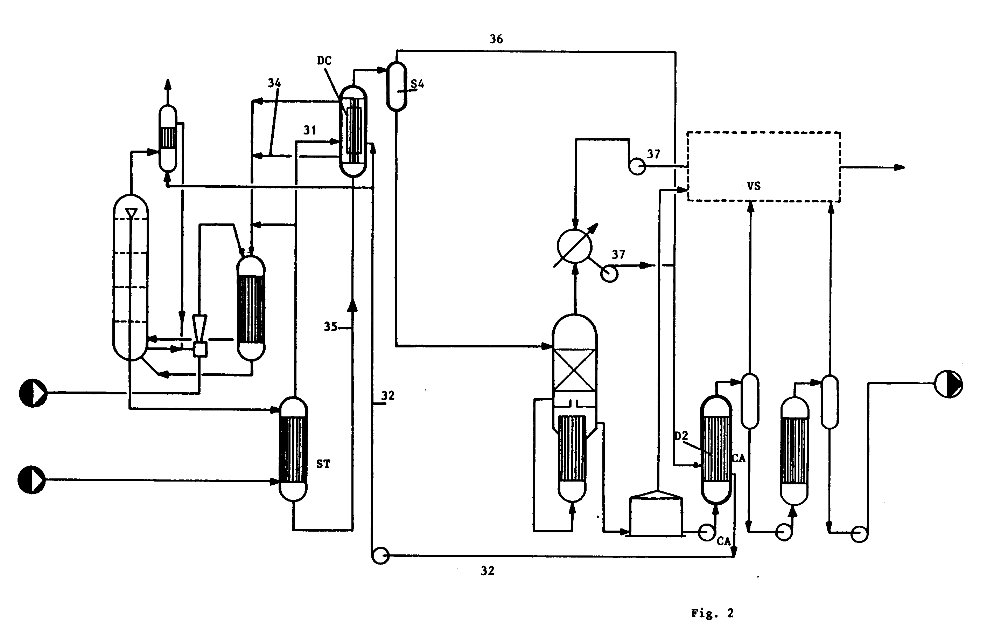

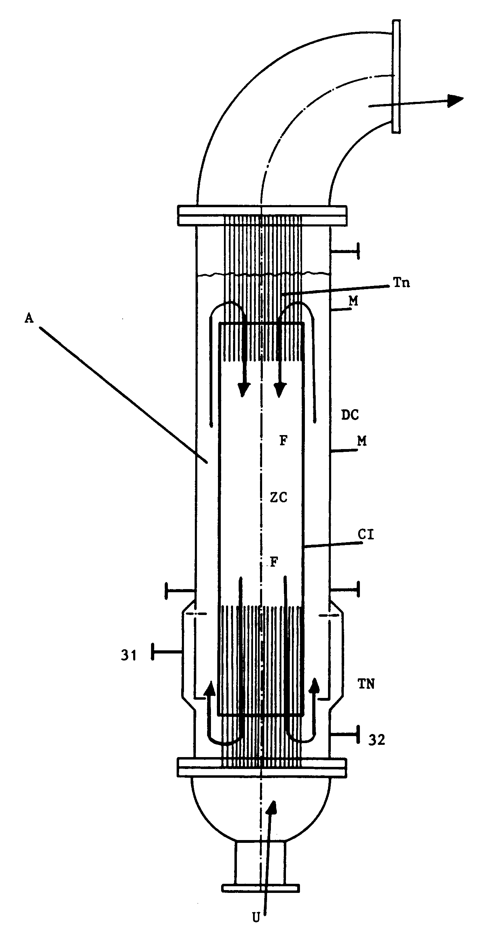

In the first type of embodiment (condensation of vapours to form carbamate, Fig. 2) a distiller-condenser DC is introduced, preferably of the type depicted in Fig. 3.

As Figure 2 shows, DC receives a part of the vapours from stripper ST (line 31) and the carbamate solution CA from the bottom of D2 through line 32. The condensate from DC goes to carbamate condenser C1 through line 34, while the urea solution from the bottom of ST is sent to be distilled through 35 in DC, followed by separator S4, whose vapours in 36 are sent to condense in D2 according to the double effect technique, known per se. In effect, the vapours from 36 (DC) are condensed in the presence of a weak carbamate solution 37 in D2 and the heat thus released is used to distill the urea solution flowing in a vacuum inside the D2 tubes.

As Figure 3 shows, it is a condenser-distiller DC in which condensation of the vapours from 31 takes place on the shell side M and the resulting heat is used to distill the urea solution flowing inside the tubes Tn.

Still in Figure 3, it can be seen that the vapours from 31 are scrubbed in an annulus of liquid A and are absorbed by the carbamate solutions 32 being heated.

Through a thermosiphon effect the return circulation (arrows F) of the solution takes place in the central zone ZC of DC, separated from the bubbling zone A by a cylinder CI open at both ends.

In this central zone ZC, the carbamate solution is cooled in counter-current transferring the heat to the urea solution U in the distillation phase flowing through tubes Tn.

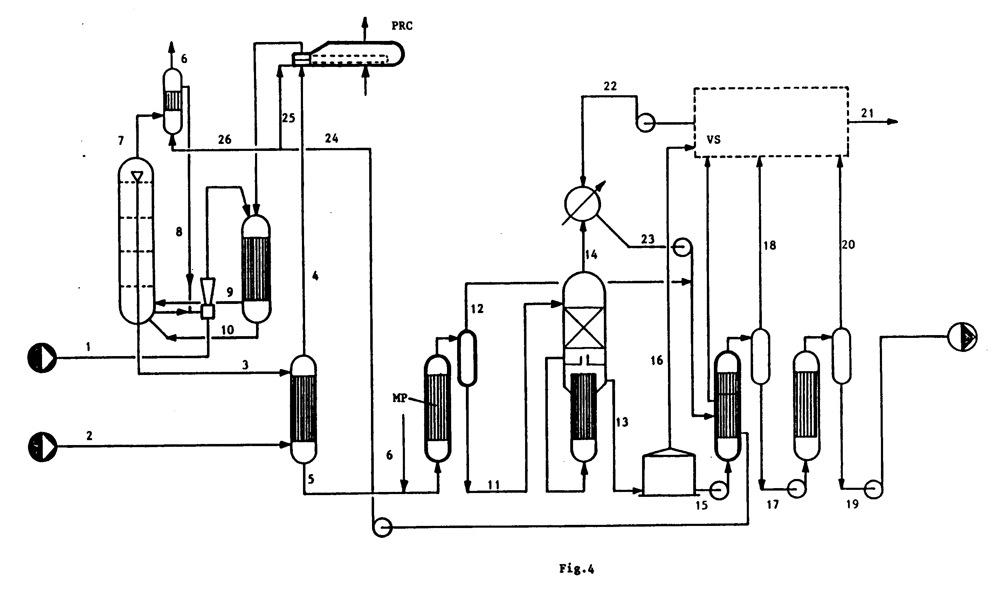

In a second form of embodiment (Fig. 4), recovery steam is produced at 6 bar abs at least, as against 4.5 bar abs in existing plants. It has been found that it is possible to produce this steam in a quantity and quality sufficient for mean pressure distillation, by introducing a pre-condenser PRC (for example of the flowing hair or mane type) to which is sent at least 80% (from line 25) of the weak recycle carbamate solution, hence of the recycle water (line 24).

With the high concentration of water in the carbamate solution so obtained, it is possible, at the same pressure, to raise the condensation heat to at least $170\,^\circ$C, thus producing the desired steam at 6 bar at least.

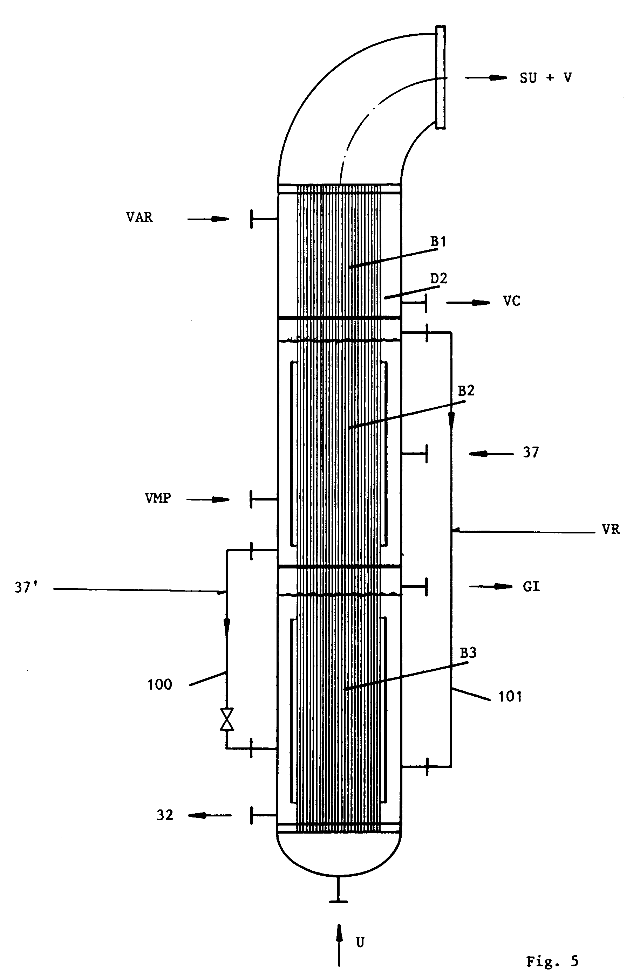

In Figure 4 too the vacuum distiller-condenser D2 is the same as that in Figure 2. Preferably, an optimized version of this has been found, permitting the maximization of the heat transfer (fig. 5).

It now consists of three sections: B1, B2 and B3. Said Figure 5 shows the whole of the apparatus which forms the vacuum distillation section (1st stage at 0.35 bar abs).

The upper part B1 operates with recovery steam VAR (from the carbamate condenser C1), whose condensate VC is discharged from the bottom of the above-mentioned zone B1, and a final concentration of 96% of urea in the distilled solution SU + V can thus be achieved.

The other part consists of two elements in series B2 and B3, in which the solution of carbamate (weak carbamate) 37 flows from top to bottom and is cooled in two distinct stages B2 and B3: in this way the final

EP 0 306 614 B1

solution of carbamate 32 has the lowest possible temperature, hence minimum pressure.

The element B3 has besides an outlet for inert gas GI in its upper part.

The carbamate solution 37′ as well as the residual vapour containing inert gas VR, are introduced, on the other hand, into ducts respectively 100 and 101 in the distiller-condenser in Figure 5.

Thanks to this pressure value (= 20 bar) the urea solution U can be distilled at mean pressure (20 bar) with recovery steam VAR at 6 bar in distiller MP.

The heat recoverable in the double effect system is not sufficient to distillate the solution up to 96% of urea. Consequently the distiller D2 is divided into two parts: an upper part B1 which uses recovery steam, and a lower part which in turn is subdivided into two zones B2 and B3, using the vapours containing inert gas VMP from the mean pressure distiller MP line 12 in Figure 4.

Operating conditions which can be achieved with the scheme described can be so summed up:
- Molar ratio NH3/CO2 in the reactor : 2.8
- Molar ratio H2O/CO2 in the reactor : 0.5
- Reactor yield : 57%
- Temperature at reactor outlet : 185°C
- Steam consumption at 20 bar : 800 kg/t
- Export recovery steam : 200 kg/t

In comparison to the reference case, a saving is achieved of 200 kg/t of steam at 20 bar, while there is a smaller recovery of 150 kg/t of steam at 4.5 bar.

With the addition of a mean pressure double-effect stage conditions can therefore be improved, even if not drastically.

In effect, the greatest obstacle to a significant improvement of the process lies in the low conversion yield of the reactor R, as a consequence of the low NH3/CO2 ratio in the reactor itself.

If the ratio is increased, this worsens operating conditions in the stripper ST, and therefore in the whole plant.

It has now been found that by adding a pre-distiller PRD upstream of the existing stripper ST the following results can be achieved:
- the possibility of feeding the stripper with a solution with a high NH3/CO2 ratio (> 2.5 weight), strippable with CO2;
- the possibility, therefore, of operating with higher NH3/CO2 ratios in the reactor, thus increasing conversion yields;
- a substantial reduction in the stripper's heat load with CO2, with ample possibilities to overload.

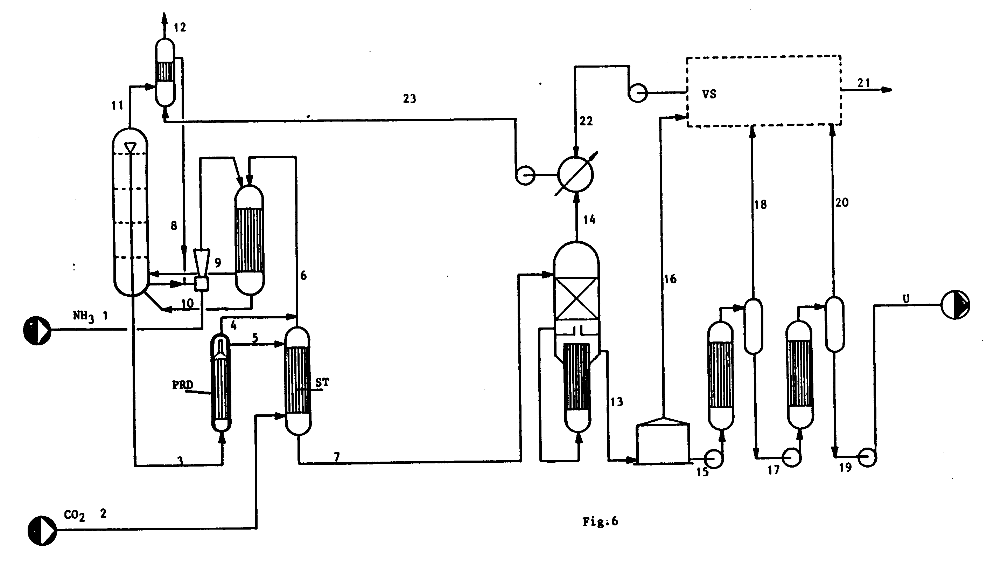

Figure 6 shows the process scheme modified by the addition of a pre-distiller. As a preliminary step, an up-flow pre-distiller has been envisaged, so as avoid modification of the driving force of circulation of the solution.

The vapours 5 leaving the pre-distiller PRD are joined to those coming from the stripper ST, while the solution of partially distilled urea flows into the existing stripper for the final stripping with CO2.

Operating conditions figures have been calculated as follows:
- Molar ratio NH3/CO2 in the reactor : 3.4
- Molar ratio H2O/CO2 in the reactor : 0.5
- Reactor yield : 64%
- Temperature at reactor outlet : 185°C
- Reactor pressure : 141 bar
- Steam consumption at 20 bar : 820 kg/t
- Export steam at 4.5 bar : 120 kg/t.

The operating conditions obtained are therefore similar to those estimated for double effect.

According to a particularly advantageous aspect of the invention, the improvements achieved thanks to the adoption of the double-effect system (Figures 2 to 5) and to the addition of a pre-distiller (Figure 6) can be combined, in the sense that their simultaneous application results substantially in the accumulation of the effects.

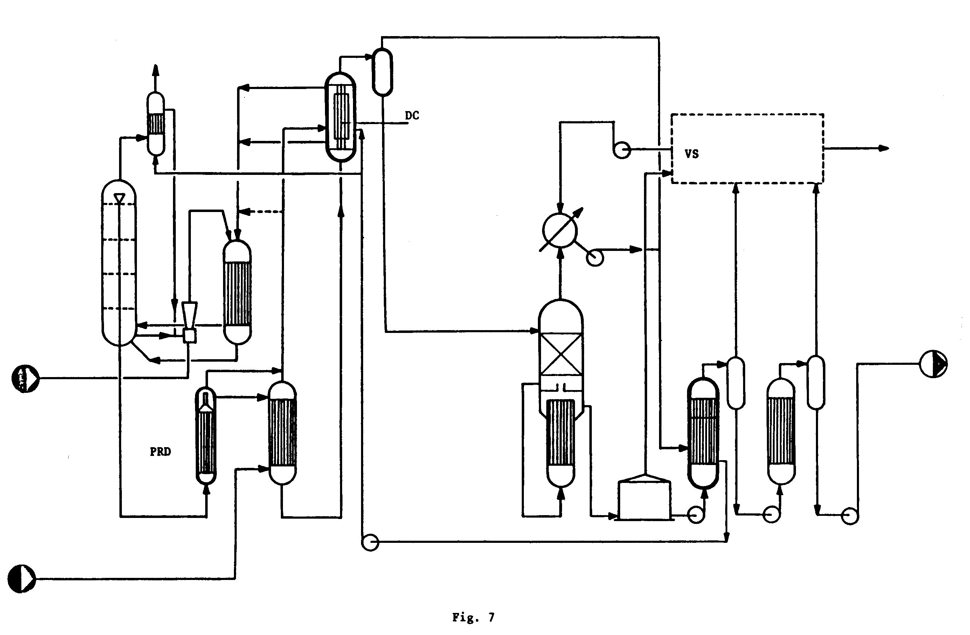

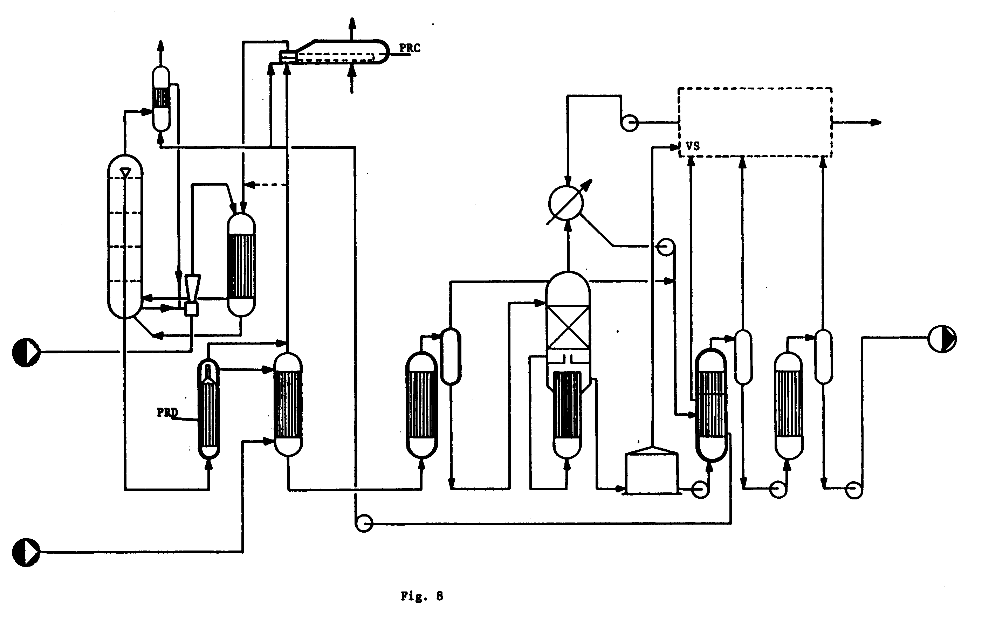

Figures 7 and 8 show the stripping process with CO2 with the addition of a pre-distiller PRD and of the double-effect system, the latter in the two variations already described, i.e. in Figure 7 with the distiller-condenser DC from Figure 2, and in Figure 8 with the pre-condenser PRC from Figure 4.

The remarkable operating conditions which can be achieved are:
- Molar ratio NH3/CO2 in the reactor : 3.4
- Molar ratio H2O/CO2 in the reactor : 0.4
- Reactor yield : 64%
- Temperature at reactor outlet : 185°C

4

- Reactor pressure : 141 bar
- Steam consumption at 20 bar : 650 kg/t
- Export recovery steam at 4.5 bar : 100 kg/t.

By way of illustration but not of limitation the following balances or examples in Tables I, II and III are set out here.

EXAMPLE 1 (Table I)      - Conventional process (Figure 1)
EXAMPLE 2 (Table II)     - Double-effect process (Figures 2 and 4)
EXAMPLE 3 (Table III)    - Process with pre-distiller (Figure 6)

CAPACITY - 1000 MTD UREA

$NH_3/CO_2$ = 2.8
$H_2O/CO_2$ = 0.4
η = 57%

CO₂ STRIPPING PROCESS

REFERENCE CASE

TABLE 1

PRELIMINARY    page 1

| LINE | 1 | | 2 | | 3 | | 4 | | 5 | | 6 | |
|---|---|---|---|---|---|---|---|---|---|---|---|---|
| Physical stage | liquid | | gas | | liquid | | vapours | | liquid | | vapours | |
| P (Kg/cm² abs.) | 160 | | 141 | | 141 | | 141 | | 141 | | 141 | |
| T (°C) | 40 | | 100 | | 183 | | 180 | | 170 | | 160 | |
| COMPOSITION | kg/h | %w | kg/h | %w | kg/h | %w | kg/h | %w | kg/h | %w | kg/h | %w |
| Urea | | | | | 43403 | 34,62 | | | 41667 | 57,10 | | |
| NH₃ | 23625 | 100,00 | | | 35814 | 28,56 | 31617 | 38,11 | 5181 | 7,10 | 1055 | 83,00 |
| CO₂ | | | 30555 | 100,00 | 24011 | 19,15 | 49133 | 59,22 | 6706 | 9,19 | 183 | 14,40 |
| H₂O | | | | | 22158 | 17,67 | 2219 | 2,67 | 19418 | 26,61 | 33 | 2,60 |
| TOTAL | 23625 | 100,00 | 30555 | 100,00 | 125386 | 100,00 | 82969 | 100,00 | 72972 | 100,00 | 1271 | 100,00 |

| LINE | 7 | | 8 | | 9 | | 10 | | 11 | | 12 | |
|---|---|---|---|---|---|---|---|---|---|---|---|---|
| Physical stage | vapours | | liquid | | vapours | | liquid | | liquid | | vapours | |
| P (Kg/cm² abs.) | 141 | | | | 141 | | 141 | | 3,5 | | 3,5 | |
| T (°C) | 183 | | | | 167 | | 167 | | 135 | | 135 | |
| COMPOSITION | kg/h | %w | kg/h | %w | kg/h | %w | kg/h | %w | kg/h | %w | kg/h | %w |
| Urea | | | | | | | | | 41667 | 69,12 | | |
| NH₃ | 5438 | 53,45 | 10619 | 36,64 | 13895 | 52,14 | 51966 | 47,71 | 793 | 1,31 | 4388 | 34,59 |
| CO₂ | 4425 | 43,50 | 11131 | 38,41 | 11990 | 44,99 | 48274 | 44,32 | 313 | 0,52 | 6393 | 50,40 |
| H₂O | 310 | 3,05 | 7228 | 24,95 | 765 | 2,87 | 8682 | 7,97 | 17514 | 29,05 | 1904 | 15,01 |
| TOTAL | 10173 | 100,00 | 28978 | 100,00 | 26650 | 100,00 | 108922 | 100,00 | 60287 | 100,00 | 12685 | 100,00 |

CAPACITY - 1000 MTD UREA

$NH_3/CO_2$ = 2,8     CO$_2$ STRIPPING PROCESS        TABLE 1        PRELIMINARY                    page 2

$H_2O/CO_2$ = 0,4     REFERENCE CASE

$\xi$ = 57 %

| LINE | 13 | | 14 | | 15 | | 16 | | 17 | | 18 | |
|---|---|---|---|---|---|---|---|---|---|---|---|---|
| Physical stage | liquid | | vapours | | liquid | | vapours | | liquid | | vapours | |
| P(Kg/cm$^2$ abs.) | 1,0 | | 1,0 | | 0,3 | | 0,3 | | 0,05 | | 0,05 | |
| T (°C) | 92 | | 100 | | 125 | | 125 | | 140 | | 140 | |
| COMPOSITION | kg/h | %w | kg/h | %w | kg/h | %w | kg/h | %w | kg/h | %w | kg/h | %w |
| Urea | 41667 | 73,08 | | | 41667 | 96,00 | | | 41667 | 99,8 | | |
| $NH_3$ | 336 | 0,99 | 457 | 13,97 | | | 336 | 2,47 | | | | |
| $CO_2$ | 114 | 0,20 | 199 | 6,08 | | | 114 | 0,84 | | | | |
| $H_2O$ | 14899 | 26,13 | 2615 | 79,95 | 1736 | 4,00 | 13163 | 96,69 | 83 | 0,2 | 1653 | 100,00 |
| TOTAL | 57016 | 100,00 | 3271 | 100,00 | 43403 | 100,00 | 13613 | 100,00 | 41750 | 100,00 | 1653 | 100,00 |

| LINE | 19 | | 20 | | 21 | | 22 | | 23 | | | |
|---|---|---|---|---|---|---|---|---|---|---|---|---|
| Physical stage | liquid | | liquid | | liquid | | | | | | | |
| P(kg/cm$^2$ abs.) | 3,5 | | 3,5 | | | | | | | | | |
| T (°C) | 50 | | 71 | | | | | | | | | |
| COMPOSITION | kg/h | %w | kg/h | %w | kg/h | %w | kg/h | %w | kg/h | %w | kg/h | %w |
| Urea | | | | | | | | | | | | |
| $NH_3$ | 1848 | 25,00 | 6236 | 31,06 | | | | | | | | |
| $CO_2$ | 496 | 6,71 | 6889 | 34,31 | | | | | | | | |
| $H_2O$ | 5047 | 68,29 | 6951 | 34,63 | 12417 | 100,00 | | | | | | |
| TOTAL | 7391 | 100,00 | 20076 | 100,00 | 12417 | 100,00 | | | | | | |

EP 0 306 614 B1

CAPACITY - 1000 MTD

NH$_3$/CO$_2$ = 2.8   DOUBLE EFFECT CASE          TABLE 2          PRELIMINARY          page 1

H$_2$O/CO$_2$ = 0,5                                inerts = 0

$\frac{}{2}$ = 57 %                                losses = 0

| LINE | 1 | | 2 | | 3 | | 4 | | 5 | | 6 | |
|---|---|---|---|---|---|---|---|---|---|---|---|---|
| Physical stage | liquid | | gas | | liquid | | vapours | | liquid | | vapours | |
| P(Kg/cm$^2$ abs.) | 160 | | 141 | | 141 | | 141 | | 141 | | 141 | |
| T (°C) | 40 | | 100 | | 185 | | 180 | | 168 | | 160 | |
| COMPOSITION | kg/h | %w | kg/h | %w | kg/h | %w | kg/h | %w | kg/h | %w | kg/h | %w |
| Urea | | | | | 43403 | 34,00 | | | 41667 | 50,45 | | |
| NH$_3$ | 23625 | 100,00 | | | 35814 | 28,05 | 28216 | 37,19 | 8672 | 10,50 | 1055 | 83,00 |
| CO$_2$ | | | 30555 | 100,00 | 24011 | 18,81 | 44690 | 59,08 | 11149 | 13,50 | 183 | 14,40 |
| H$_2$O | | | | | 24443 | 19,14 | 2822 | 3,73 | 21100 | 25,55 | 33 | 2,60 |
| TOTAL | 23625 | 100,00 | 30555 | 100,00 | 127671. | 100,00 | 75638 | 100,00 | 82588 | 100,00 | 1271 | 100,00 |

| LINE | 7 | | 8 | | 9 | | 10 | | 11 | | 12 | |
|---|---|---|---|---|---|---|---|---|---|---|---|---|
| Physical stage | vapours | | | | | | | | liquid | | vapours | |
| P(kg/cm$^2$ abs.) | 141 | | | | | | | | 19 | | 19 | |
| T (°C) | 185 | | | | | | | | 156 | | 159 | |
| COMPOSITION | kg/h | %w | kg/h | %w | kg/h | %w | kg/h | %w | kg/h | %w | kg/h | %w |
| Urea | | | | | | | | | 41667 | 58,95 | | |
| NH$_3$ | 5438 | 53,45 | | | | | | | 5654 | 8,00 | 3018 | 25,34 |
| CO$_2$ | 4425 | 43,50 | | | | | | | 3887 | 5,50 | 7262 | 60,97 |
| H$_2$O | 310 | 3,05 | | | | | | | 19470 | 25,50 | 1630 | 13,69 |
| TOTAL | 10173 | 100,00 | | | | | | | 70678 | 100,00 | 11910 | 100,00 |

EP 0 306 614 B1

CAPACITY - 1000 MTD

NH$_3$/CO$_2$ = 2,8  DOUBLE EFFECT CASE  TABLE 2  PRELIMINARY  page 2

H$_2$O/CO$_2$ = 0,5

$\zeta$ = 57 %  inerts =0

losses =0

| LINE | 13 | | 14 | | 15 | | 16 | | 17 | | 18 | |
|---|---|---|---|---|---|---|---|---|---|---|---|---|
| Physical stage | liquid | | vapours | | liquid | | vapours | | liquid | | vapours | |
| P(Kg/cm$^2$ abs.) | 3,5 | | 3,5 | | 1 | | 1 | | 0,3 | | 0,3 | |
| T (°C) | 135 | | 135 | | 92 | | 92 | | 125 | | 125 | |
| COMPOSITION | kg/h | %w | kg/h | %w | kg/h | %w | kg/h | %w | kg/h | %w | kg/h | %w |
| Urea | 41667 | 69,12 | | | 41667 | 73,08 | | | 41667 | 96,00 | | |
| NH$_3$ | 793 | 1,31 | 4861 | 46,78 | 336 | 0,59 | 457 | 13,97 | | | 336 | 2,47 |
| CO$_2$ | 313 | 0,52 | 3574 | 34,40 | 114 | 0,20 | 199 | 6,08 | | | 114 | 0,84 |
| H$_2$O | 17514 | 29,05 | 1956 | 18,82 | 14899 | 26,13 | 2615 | 79,95 | 1736 | 4,00 | 13163 | 96,69 |
| TOTAL | 60287 | 100,00 | 10391 | 100,00 | 57016 | 100,00 | 3271 | 100,00 | 43403 | 100,00 | 13613 | 100,00 |

| LINE | 19 | | 20 | | 21 | | 22 | | 23 | | 24 | |
|---|---|---|---|---|---|---|---|---|---|---|---|---|
| Physical stage | liquid | | vapours | | liquid | | liquid | | liquid | | liquid | |
| P(kg/cm$^2$ abs.) | 0,05 | | 0,05 | | | | 3,5 | | 3,5 | | 141 | |
| T (°C) | 140 | | 140 | | | | 50 | | 40 | | 120 | |
| COMPOSITION | kg/h | %w | kg/h | %w | kg/h | %w | kg/h | %w | kg/h | %w | kg/h | %w |
| Urea | 41667 | 99,8 | | | | | | | | | | |
| NH$_3$ | | | | | | | 793 | 12,96 | 5654 | 34,24 | 9727 | 32,76 |
| CO$_2$ | 83 | 0,2 | | | | | 313 | 5,11 | 3887 | 23,54 | 11332 | 38,17 |
| H$_2$O | | | 1653 | 100,00 | 12417 | 100,00 | 5014 | 81,93 | 6970 | 42,22 | 8633 | 29,07 |
| TOTAL | 41750 | 100,00 | 1653 | 100,00 | 12417 | 100,00 | 6120 | 100,00 | 16511 | 100,00 | 29692 | 100,00 |

EP 0 306 614 B1

CAPACITY — 1000 MTD

$NH_3/CO_2$ = 2,8

$H_2O/CO_2$ = 0,5    DOUBLE EFFECT CASE                    TABLE 2            PRELIMINARY        page 3

$\frac{?}{?}$ = 57 %                                                                 inerts = 0
                                                                                    losses = 0

| LINE | 25 | | 26 | | | | | | | | | |
|---|---|---|---|---|---|---|---|---|---|---|---|---|
| Physical stage | liquid | | liquid | | | | | | | | | |
| P($Kg/cm^2$ abs.) | 141 | | 141 | | | | | | | | | |
| T (°C) | 120 | | 120 | | | | | | | | | |
| COMPOSITION | kg/h | %w | kg/h | %w | kg/h | %w | kg/h | %w | kg/h | %w | kg/h | %w |
| Urea | | | | | | | | | | | | |
| $NH_3$ | 8249 | 32,76 | 1478 | 32,75 | | | | | | | | |
| $CO_2$ | 9611 | 38,17 | 1721 | 38,13 | | | | | | | | |
| $H_2O$ | 7319 | 29,07 | 1314 | 29,12 | | | | | | | | |
| TOTAL | 25179 | 100,00 | 4513 | 100,00 | | | | | | | | |

| LINE | | | | | | | | | | | | |
|---|---|---|---|---|---|---|---|---|---|---|---|---|
| Physical stage | | | | | | | | | | | | |
| P($kg/cm^2$ abs.) | | | | | | | | | | | | |
| T (°C) | | | | | | | | | | | | |
| COMPOSITION | kg/h | %w | kg/h | %w | kg/h | %w | kg/h | %w | kg/h | %w | kg/h | %w |
| Urea | | | | | | | | | | | | |
| $NH_3$ | | | | | | | | | | | | |
| $CO_2$ | | | | | | | | | | | | |
| $H_2O$ | | | | | | | | | | | | |
| TOTAL | | | | | | | | | | | | |

EP 0 306 614 B1

CAPACITY - 1000 MTD UREA

NH₃/CO₂ = 3.4   CASE WITH PREDISTILLER    TABLE 3    PRELIMINARY    page 1
H₂O/CO₂ = 0.5   AND STRIPPER
∮ = 64 %                                              inerts = 0
                                                     losses = 0

| LINE | 1 | | 2 | | 3 | | 4 | | 5 | | 6 | |
|---|---|---|---|---|---|---|---|---|---|---|---|---|
| Physical stage | liquid | | gas | | liquid | | vapours | | liquid | | vapours | |
| P(Kg/cm² abs.) | 160 | | 141 | | 141 | | 141 | | 141 | | 141 | |
| T (°C) | 40 | | 100 | | 185 | | 200 | | 200 | | 200 | |
| COMPOSITION | kg/h | %w | kg/h | %w | kg/h | %w | kg/h | %w | kg/h | %w | kg/h | %w |
| Urea | | | | | 43403 | 34,66 | | | 43403 | 39,13 | | |
| NH₃ | 23625 | 100,00 | | | 40736 | 32,53 | 8307 | 58,00 | 32429 | 29,24 | 27353 | 41,63 |
| CO₂ | | | 30555 | 100,00 | 17904 | 14,29 | 5586 | 39,00 | 12318 | 11,11 | 36570 | 55,65 |
| H₂O | | | | | 23194 | 18,52 | 430 | 3,00 | 22764 | 20,52 | 1789 | 2,72 |
| TOTAL | 23625 | 100,00 | 30555 | 100,00 | 125237 | 100,00 | 14323 | 100,00 | 110914 | 100,00 | 65712 | 100,00 |

| LINE | 7 | | 8 | | 9 | | 10 | | 11 | | 12 | |
|---|---|---|---|---|---|---|---|---|---|---|---|---|
| Physical stage | liquid | | | | | | | | vapours | | vapours | |
| P(kg/cm² abs.) | 141 | | | | | | | | 141 | | 141 | |
| T (°C) | 170 | | | | | | | | 185 | | 160 | |
| COMPOSITION | kg/h | %w | kg/h | %w | kg/h | %w | kg/h | %w | kg/h | %w | kg/h | %w |
| Urea | 41667 | 55,00 | | | | | | | | | | |
| NH₃ | 6060 | 8,00 | | | | | | | 5438 | 53,45 | 1055 | 83,00 |
| CO₂ | 7576 | 10,00 | | | | | | | 4425 | 43,50 | 183 | 14,40 |
| H₂O | 20454 | 27,00 | | | | | | | 310 | 3,05 | 33 | 2,60 |
| TOTAL | 75757 | 100,00 | | | | | | | 10173 | 100,00 | 1271 | 100,00 |

EP 0 306 614 B1

CAPACITY - 1000 MTD UREA  CASE WITH PREDISTILLER AND STRIPPER

NH3/CO2 = 3,4
H2O/CO2 = 0,5
$\}$ = 64 %

TABLE 3

PRELIMINARY

inerts = 0
losses = 0

page 2

| LINE | 13 kg/h | 13 Zw | 14 kg/h | 14 Zw | 15 kg/h | 15 Zw | 16 kg/h | 16 Zw | 17 kg/h | 17 Zw | 18 kg/h | 18 Zw |
|---|---|---|---|---|---|---|---|---|---|---|---|---|
| Physical stage | liquid | | vapours | | liquid | | vapours | | liquid | | vapours | |
| P (kg/cm² abs.) | 3,5 | | 3,5 | | 1,0 | | 1 | | 0,3 | | 0,3 | |
| T (°C) | 135 | | 135 | | 92 | | 100 | | 125 | | 125 | |
| COMPOSITION | kg/h | Zw | kg/h | Zw | kg/h | Zw | kg/h | Zw | kg/h | Zw | kg/h | Zw |
| Urea | 41667 | 69,12 | | | 41667 | 73,08 | | | 41667 | 96,00 | | |
| NH3 | 793 | 1,31 | 5267 | 34,05 | 336 | 0,59 | 457 | 13,97 | | | 336 | 2,47 |
| CO2 | 313 | 0,52 | 7263 | 46,95 | 114 | 0,20 | 199 | 6,08 | | | 114 | 0,84 |
| H2O | 17514 | 29,05 | 2940 | 19,00 | 14899 | 26,13 | 2615 | 79,95 | 1736 | 4,00 | 13163 | 96,69 |
| TOTAL | 60287 | 100,00 | 15470 | 100,00 | 57016 | 100,00 | 3271 | 100,00 | 43403 | 100,00 | 13613 | 100,00 |

| LINE | 19 kg/h | 19 Zw | 20 kg/h | 20 Zw | 21 kg/h | 21 Zw | 22 kg/h | 22 Zw | 23 kg/h | 23 Zw | 24 |
|---|---|---|---|---|---|---|---|---|---|---|---|
| Physical stage | liquid | | vapours | | liquid | | liquid | | liquid | | |
| P (kg/cm² abs.) | 0,05 | | 0,05 | | - | | 3,5 | | 141 | | |
| T (°C) | 140 | | 140 | | - | | 50 | | 71 | | |
| COMPOSITION | kg/h | Zw | kg/h | Zw | kg/h | Zw | kg/h | Zw | kg/h | Zw | |
| Urea | 41667 | 99,8 | | | | | | | | | |
| NH3 | | | | | | | 1848 | 25,00 | 7115 | 31,12 | |
| CO2 | | | | | | | 496 | 6,71 | 7759 | 33,94 | |
| H2O | 83 | 0,2 | 1653 | 100,00 | 12417 | 100,00 | 5047 | 68,29 | 7987 | 34,94 | |
| TOTAL | 41750 | 100,00 | 1653 | 100,00 | 12417 | 100,00 | 7391 | 100,00 | 22861 | 100,00 | |

## Claims

1. Process for the production of urea comprising at least: a stage wherein ammonia and carbon dioxide are reacted in a reactor at a pressure of between 120 and 220 bar and temperature of between 170°C and 200°C; a stripping stage wherein the urea solution leaving the reactor and containing carbamate and water and other products, reacted or unreacted, undergoes a process to strip it of carbamate and NH3 by means of the carbon dioxide feeding the reactor; a stage wherein the stripped vapours are condensed and two vacuum distillation stages, characterized by the fact that it comprises at least one additional stage selected from the group consisting of: a mean pressure distillation stage using in

11

substance recovery heat, by preference of the double-effect type, a pre-distillation stage and a second stripping stage.

2. Process according to claim 1, characterized by the fact that the distillation heat is produced by direct condensation of the vapours forming carbamate in a condenser-distiller wherein vapour condensation takes place on the shell side, and the heat produced is used to distill the urea solution flowing inside the tubes.

3. Process according to claim 2, characterized by the fact that the vapours bubble in an annulus of liquid and are absorbed by the carbamate solution which is thereby heated, and by thermosiphon effect the solution flows by return circulation into a central zone where the carbamate solution is cooled in counter-current transferring heat to the solution.

4. Process according to claim 1, characterized by the fact that recovery vapour is produced at 6 bar abs at least in a quantity and of a quality sufficient for distillation.

5. Process according to claim 4, characterized by the fact that a pre-condenser is used, into which is directed at least 80% of the carbamate solution, so that the condensation heat can be raised to at least 170 °C thus making it possible to produce vapour at 6 bar abs at least.

6. Process according to claims 2 and 5, characterized by the fact that a distiller-condenser in three superimposed sections is used, in which the urea solution is concentrated on the tube side up to 92÷97% weight at the upper section outlet, whereas on the shell side the heat is provided in the upper section by condensing the recovery vapour, and in the intermediate and lower sections by condensing vapours to form carbamate in two distinct sections in counter-current to the urea solution so that the condensation of the above vapours is effected at the minimum possible temperature and pressure.

7. Process according to claim 1, characterized by the fact that a pre-distiller is used.

8. Process according to claim 1, characterized by the combination of a pre-distiller and of a double-effect distiller-condenser.

**Patentansprüche**

1. Verfahren zur Herstellung von Harnstoff, das wenigstens
   - eine Stufe, bei welcher man Ammoniak und Kohlendioxid in einem Reaktor bei einem Druck zwischen 120 und 220 bar und einer Temperatur zwischen 170 °C und 200 °C reagieren läßt,
   - eine Abstreifstufe, bei welcher die Harnstofflösung, die den Reaktor verläßt und Carbamat, Wasser und andere Produkte enthält, die reagiert haben oder nicht, einem Prozeß unterzogen wird, um von ihr mit Hilfe des dem Reaktor zugeführten Kohlendioxids Carbamat und $NH_3$ abzustreifen,
   - eine Stufe, bei welcher die abgestreiften Dämpfe kondensiert werden, und
   - zwei Vakuumdestillationsstufen aufweist,
     dadurch gekennzeichnet, daß es wenigstens eine zusätzliche Stufe aufweist, die aus der Gruppe gewählt wird, die aus
   - einer Mitteldruck-Destillationsstufe, vorzugsweise in Zweistufenbauweise, bei der im wesentlichen Rückgewinnungswärme verwendet wird,
   - einer Vordestillationsstufe und
   - einer zweiten Abstreifstufe besteht.

2. Verfahren nach Anspruch 1, dadurch gekennzeichnet, daß die Destillationswärme durch direkte Kondensation der Carbamat bildenden Dämpfe in einem Kondensator-Destillator erzeugt wird, wobei Dampfkondensation auf der Schalenseite stattfindet und die erzeugte Wärme verwendet wird, um die im Inneren der Rohre fließende Harnstofflösung zu destillieren.

3. Verfahren nach Anspruch 2, dadurch gekennzeichnet,
   - daß die Dämpfe als Blasen in einem Flüssigkeitsring vorhanden sind und von der Carbamatlösung absorbiert werden, die dadurch erwärmt wird, und

EP 0 306 614 B1

- daß durch Thermosiphonwirkung die Lösung durch Rückführumwälzung in einen zentralen Bereich fließt, wo die Carbamatlösung im Gegenstrom gekühlt wird, wobei Wärme auf die Lösung übertragen wird.

4. Verfahren nach Anspruch 1, dadurch gekennzeichnet, daß Rückgewinnungsdampf von 6 bar absolut wenigstens in einer Menge und von einer Qualität erzeugt wird, die für eine Destillation ausreicht.

5. Verfahren nach Anspruch 4, dadurch gekennzeichnet, daß ein Vorkondensator verwendet wird, in den wenigstens 80% der Carbamatlösung gerichtet werden, so daß die Kondensationswärme auf wenigstens 170 °C angehoben werden kann, wodurch die Erzeugung von Dampf mit wenigstens 6 bar absolut ermöglicht wird.

6. Verfahren nach den Ansprüchen 2 und 5, dadurch gekennzeichnet, daß ein Destillator-Kondensator in drei übereinanderliegenden Abschnitten verwendet wird, bei welchem die Harnstofflösung auf der Rohrseite auf bis zu 92 bis 97 Gewichtsprozent an dem Auslaß des oberen Abschnitts konzentriert wird, während auf der Schalenseite die Wärme in dem oberen Abschnitt durch Kondensieren des Rückgewinnungsdampfes, in dem Zwischenabschnitt und in dem unteren Abschnitt durch Kondensieren von Dämpfen zur Bildung von Carbamat in zwei verschiedenen Abschnitten im Gegenstrom zu der Harnstofflösung bereitgestellt wird, so daß die Kondensation der obigen Dämpfe bei kleinstmöglicher Temperatur und kleinstmöglichem Druck bewirkt wird.

7. Verfahren nach Anspruch 1, dadurch gekennzeichnet, daß ein Vordestillator verwendet wird.

8. Verfahren nach Anspruch 1, gekennzeichnet durch die Kombination aus einem Vordestillator und aus einem zweistufigen Destillator-Kondensator.

**Revendications**

1. Procédé de production d'urée comprenant au moins une étape dans laquelle de l'ammoniac et du dioxyde de carbone réagissent dans un réacteur à une pression comprise entre 120 et 220 bar et une température comprise entre 170°C et 200°C; une étape d'entraînement où la solution d'urée quittant le réacteur et contenant le carbamate et l'eau et les autres produits, ayant réagi ou n'ayant pas réagi, subissent un procédé au cours duquel il est éliminé du carbamate et du $NH_3$ au moyen de dioxyde de carbone alimentant le réacteur; une étape au cours de laquelle les vapeurs entraînées par extraction sont condensées et deux étapes de distillation sous vide, caractérisé par le fait qu'il comprend au moins une étape additionnelle choisie dans le groupe consistant en: une étape de distillation sous pression moyenne faisant emploi substantiellement de récupération de chaleur, de préférence du type à double effet, une étape de pré-distillation et une deuxième étape d'entraînement.

2. Procédé selon la revendication 1, caractérisé en ce que la chaleur de distillation est produite par condensation directe des vapeurs du carbamate dans un appareil de condensation-distillation dans lequel la condensation de vapeur se produit sur le côté de la paroi et la chaleur produite est utilisée pour distiller la solution d'urée circutant à l'intérieur des tubes.

3. Procédé selon la revendication 2, caractérisé en ce que les vapeurs barbotent dans un anneau de liquide et sont absorbées par la solution de carbamate qui est par conséquent chauffée et, par un effet de thermosiphon, la solution passe par circulation retour dans une zone centrale dans laquelle la solution de carbamate est refroidie à contre-courant en transférant sa chaleur à la solution.

4. Procédé selon la revendication 1, caractérisé en ce que la vapeur récupérée est produite à une pression de 6 bar absolu au moins en une quantité et en une qualité suffisantes pour permettre la distillation.

5. Procédé selon la revendication 4, caractérisé en ce que l'on emploie un pré-condenseur dans lequel est introduit au moins 80% de la solution de carbamate de sorte que la chaleur de condensation puisse être portée à au moins 170°C de façon à rendre par conséquent possible la production de vapeur à 6 bar absolus au moins.

13

6. Procédé selon les revendications 2 et 5, caractérisé en ce que l'on emploie un appareil de distillation-condensation formé de trois sections superposées dans lesquelles la solution durée est concentrée sur la paroi supérieure du côté tube jusqu'à 92-97% en poids à la sortie de la section supérieure de sorte que du côté paroi la chaleur soit fournie à la section supérieure par condensation de la vapeur de récupération et en ce que dans les sections intermédiaire et basse, par condensation de vapeur, on forme du carbamate en deux sections distinctes à contre-courant de la solution d'urée de sorte que la condensation des vapeurs soit effectuée aux pression et température les plus basses possibles.

7. Procédé selon la revendication 1, caractérisé en ce que l'on utilise un appareil de pré-distillation.

8. Procédé selon la revendication 1, caractérisé en ce qu'il comporte la combinaison d'un appareil de prédistillation et d'un appareil de distillation-condensation à double effet.

Fig. 1

Fig. 2

Fig. 3

Fig.4

Fig. 5

Fig.6

Fig. 7

Fig. 8